# EUROPEAN PATENT APPLICATION

(11) **EP 4 706 665 A1**
(43) Date of publication of application: **11.03.2026**
(21) Application number: 24802690.8
(22) Date of filing: 12.04.2024
(51) Int. Cl.: A61K 33/08, A61P 19/02, A61K 9/127, A61K 9/133

(54) **MAGNESIUM HYDROXIDE NANOPARTICLES FOR TREATING ARTHRALGIA, AND PREPARATION METHOD THEREFOR AND USE THEREOF**

(30) Priority: 05.05.2023 CN 202310496323; 05.05.2023 CN 202310497579
(71) Applicant: Xiangya Hospital, Central South University, Changsha, Hunan 410008 (CN)
(72) Inventor: LEI, Guanghua, Changsha, Hunan 410008 (CN); ZENG, Chao, Changsha, Hunan 410008 (CN); DENG, Caifeng, Changsha, Hunan 410008 (CN)
(74) Representative: Vesterinen, Jussi Tapio
(86) International application number: PCT/CN2024/087375
(87) International publication number: WO 2024/230404

(57) **Abstract**

Provided are magnesium hydroxide nanoparticles for treating joint pain, a preparation method therefor, and application thereof, relating to the pharmaceutical field. The preparation method includes: dissolving soluble magnesium salt in purified water to obtain a magnesium salt solution; dissolving soluble metal hydroxide in purified water to obtain a metal hydroxide solution; dissolving phospholipid in an organic solvent to obtain a phospholipid solution; carrying out rotary evaporation on the phospholipid solution to remove the organic reagent, and after a thin film is formed, adding the magnesium salt solution for hydration to obtain a hydration solution; and adding the metal hydride solution into the hydration solution for nanonization treatment, and obtaining magnesium hydroxide nanoparticles upon the completion of nanonization treatment. The preparation method is simple and easy to control, and magnesium hydroxide nanoparticles can be prepared only by magnesium salt, hydroxide and phospholipid. Moreover, rat experiments verify that the magnesium salt prepared into nano-scale magnesium hydroxide through pharmaceutical preparation technology exhibits joint analgesic efficacy, and the analgesic efficacy is significantly prolonged, which can greatly reduce a dosing frequency and further reduce the pain of a patient.

## Description

### CROSS-REFERENCE TO RELATED APPLICATION

This patent application claims the priority of Chinese Patent Application No. 202310496323.3 filed with the China National Intellectual Property Administration on May, 05, 2023 and entitled "Magnesium hydroxide nanoparticles for treating joint pain, preparation method therefor and application thereof", the disclosure of which is incorporated herein by reference in its entirety.

This patent application claims the priority of Chinese Patent Application No. 202310497579.6 filed with the China National Intellectual Property Administration on May, 05, 2023 entitled "Application of magnesium hydroxide in preparation of medicine for treating joint pain", the disclosure of which is incorporated herein by reference in its entirety.

### TECHNICAL FIELD

The present disclosure belongs to the field of medicines, and in particular to magnesium hydroxide nanoparticles for treating joint pain, a preparation method therefor, and application thereof.

### BACKGROUND

Osteoarthritis (OA) is a degenerative disease caused by multiple factors, such as fibrosis, cracking, ulceration, and loss of articular cartilage, with joint pain as the main symptom. OA often affects sites such as knee joints, hip joints, spine, and hands, with pathological features mainly including degeneration and destruction of articular cartilage, subchondral bone sclerosis or cyst formation, osteophyte formation at joint margins, and synovitis. OA seriously affects joint function and quality of life of the patient, and it is found that OA, particularly symptomatic knee OA, is significantly associated with an increased risk of all-cause mortality. Unfortunately, there is no safe and effective treatment method in China and at abroad to delay the progress of OA.

Joint pain is the most common clinical manifestation of OA, so pain relief is the primary reason for the patient with OA to seek medical care. With the progress of the disease, the patient with OA often experiences persistent pain. Therefore, achieving long-acting and safe analgesia is of great significance for the patient with OA.

At present, treatment methods for the patient with OA are primarily focused on joint pain relief. Although there is a wide variety of treatment methods, there are still numerous issues. For example, acetaminophen used to be the first-line drug to treat OA pain, but in recent years, high-quality research evidence shows that the acetaminophen is not only ineffective in relieving OA pain but also significantly increases the risk of gastrointestinal side effects compared with placebo. Oral non-steroidal anti-inflammatory drugs have a clear effect on relieving OA pain, but the cardiovascular and gastrointestinal side effects associated with chronic administration have raised safety concerns. Oral tramadol is recommended as a "first-line drug" by 2013 American Academy of Orthopaedic Surgeons and 2012 American College of Rheumatology Clinical Practice Guidelines for OA. However, in recent years, it has been found that oral tramadol can significantly increase all-cause mortality and the incidence of myocardial infarction and hip fracture compared with non-steroidal anti-inflammatory drugs (it has been written into 2019 American College of Rheumatology Clinical Practice Guidelines for OA and tramadol is no longer recommended as a "first-line drug"). Intra-articular injection of hormones is widely used and has obvious short-term pain relief effect, but there are some important problems to be solved urgently in the intra-articular injection of hormones, such as short duration of pain relief effect, insufficient effect and potential safety hazards. Therefore, there is an urgent need to explore a therapeutic drug for OA that offers significant efficacy, high safety profile, and provide long-acting analgesic effects.

Based on this, the technical solution of the present disclosure is proposed.

### SUMMARY

To solve the problem in the prior art, the present disclosure provides magnesium hydroxide nanoparticles for treating joint pain and a preparation method therefor. The preparation method is simple and easy to control, and magnesium hydroxide nanoparticles can be prepared only by magnesium salt, hydroxide and phospholipid. Moreover, rat experiments verify that the magnesium salt prepared into nano-scale magnesium hydroxide through pharmaceutical preparation technology exhibits joint analgesic efficacy, and the analgesic efficacy is significantly prolonged, which can greatly reduce a dosing frequency and further reduce the pain of a patient.

The solution of the present disclosure is as follows: magnesium hydroxide nanoparticles for treating joint pain are provided, where the magnesium hydroxide nanoparticles include magnesium hydroxide and phospholipid.

Preferably, the magnesium hydroxide nanoparticle includes a "core-shell" structure, in which
a core layer is magnesium hydroxide, and a shell layer is phospholipid.

Preferably, the phospholipid is one or more of natural soybean phospholipid, natural egg yolk phospholipid, and synthetic phospholipid (such as hydrogenated phospholipid).

Preferably, the phospholipid is one or a combination of multiple phospholipids selected from S45, S75, S100, SPC, E80, EPCS, EPG, SPC-3, PC98-T, PL-100M, HSPC, PGE, PGSH, DS-PL95E, DSPE, DPPA, DSPA, and DMPC.

Preferably, the magnesium hydroxide nanoparticles have an average particle size of 50-550 nm.

Based on the same technical concept, the present disclosure further provides a preparation method for magnesium hydroxide nanoparticles for treating joint pain, where the preparation method includes the following steps:
(1) dissolving soluble magnesium salt in purified water to obtain a magnesium salt solution;
(2) dissolving soluble metal hydroxide in purified water to obtain a metal hydroxide solution;
(3) dissolving phospholipid in an organic solvent to obtain a phospholipid solution;
(4) carrying out rotary evaporation on the phospholipid solution to remove the organic reagent, and after a thin film is formed, adding the magnesium salt solution for hydration to obtain a hydration solution; and
(5) adding the metal hydride solution into the hydration solution for nanonization treatment, and obtaining magnesium hydroxide nanoparticles for treating joint pain upon the completion of nanonization treatment.

Preferably, in step (1), in step (1), the soluble magnesium salt is one or a combination of multiple selected from anhydrous magnesium sulfate, magnesium sulfate monohydrate, magnesium sulfate heptahydrate, magnesium chloride, and magnesium chloride hexahydrate.

Preferably, in step (2), the metal hydroxide is one or a combination of sodium hydroxide and potassium hydroxide.

Preferably, in step (3), the organic solvent is volatile and can dissolve phospholipid, which is one or a combination of multiple selected from ethanol, dichloromethane, and acetone.

Preferably, in step (5), a nanonization treatment mode is one of probe ultrasonic treatment, high-pressure homogenization treatment, or ball mill treatment.

Based on the same technical concept, the present disclosure further provides application of magnesium hydroxide nanoparticles for treating joint pain, where the magnesium hydroxide nanoparticles are used for preparing a medicine for treating joint pain of osteoarthritis.

The present disclosure has beneficial effects as follows:
The preparation method is simple and easy to control, and magnesium hydroxide nanoparticles can be prepared only by magnesium salt, hydroxide and phospholipid. Moreover, rat experiments verify that the magnesium salt prepared into nano-scale magnesium hydroxide through pharmaceutical preparation technology exhibits joint analgesic efficacy, and the analgesic efficacy is significantly prolonged, which can greatly reduce a dosing frequency and further reduce the pain of a patient.

### BRIEF DESCRIPTION OF THE DRAWINGS

To describe the technical solutions in the embodiments of the present disclosure or in the prior art more clearly, the following briefly introduces the accompanying drawings required for describing the embodiments. Apparently, the accompanying drawings in the following description show merely some embodiments of the present disclosure, and a person of ordinary skill in the art may still derive other drawings from these accompanying drawings without creative efforts.
FIG. 1 is a TEM (Transmission electron microscopy) picture of magnesium hydroxide nanoparticles in Test Example 1;
FIG. 2 is a data plot showing mechanical paw withdrawal threshold results for each group of rats from Test Example 2;
FIG. 3 is a data plot showing bipedal weight bearing difference results for each group of rats from Test Example 2.

### DETAILED DESCRIPTION OF THE EMBODIMENTS

To make the objective, technical solutions and advantages of the present disclosure more clearly, the technical solutions of the present disclosure are described in detail below. Apparently, the described embodiments are merely a part rather than all of the embodiments of the present disclosure. All other implementations obtained by a person of ordinary skill in the art based on the embodiments of the present disclosure without creative efforts shall fall within the scope of protection of the present disclosure.

### Embodiment 1

This embodiment provides a preparation method for magnesium hydroxide nanoparticles for treating joint pain, where the preparation method includes the following steps.
(1) 393.6 mg of magnesium sulfate heptahydrate is mixed and dissolved with 4 mL of purified water to obtain a magnesium sulfate solution.
(2) 8 mg of sodium hydroxide is mixed and dissolved with 1 mL of purified water to obtain a sodium hydroxide solution.
(3) 125 mg of egg yolk phospholipid E80 is dissolved in 20 mL of dichloromethane to obtain a phospholipid solution.
(4) The phospholipid solution is transferred into a round-bottom flask, an organic solvent is removed by using a rotary evaporator to form a thin film on a flask wall, and then the thin film is hydrated with the magnesium sulfate solution to obtain a hydration solution.
(5) The sodium hydroxide solution is rapidly added into the hydration solution to obtain a mixture, then the mixture is sonicated with a probe at 220 W for 10 minutes; the magnesium hydroxide nanoparticles for treating joint pain are obtained upon completion of sonication; and it is determined by a laser particle size analyzer that an average particle size of the magnesium hydroxide nanoparticles is 338.5 nm.

### Embodiment 2

This embodiment provides a preparation method for magnesium hydroxide nanoparticles for treating joint pain, where the preparation method includes the following steps.
(1) 393.6 mg of magnesium sulfate heptahydrate is mixed and dissolved with 4 mL of purified water to obtain a magnesium sulfate solution.
(2) 12 mg of sodium hydroxide is mixed and dissolved with 1 mL of purified water to obtain a sodium hydroxide solution.
(3) 125 mg of egg yolk phospholipid E80 is dissolved in 30 mL of dichloromethane to obtain a phospholipid solution.
(4) The phospholipid solution is transferred into a round-bottom flask, an organic solvent is removed by using a rotary evaporator to form a thin film on a flask wall, and then the thin film is hydrated with the magnesium sulfate solution to obtain a hydration solution.
(5) The sodium hydroxide solution is rapidly added into the hydration solution to obtain a mixture, then the mixture is sonicated with a probe at 220 W for 10 minutes; the magnesium hydroxide nanoparticles for treating joint pain are obtained upon completion of sonication; and it is determined by a laser particle size analyzer that an average particle size of the magnesium hydroxide nanoparticles is 445.0 nm.

### Embodiment 3

This embodiment provides a preparation method for magnesium hydroxide nanoparticles for treating joint pain, where the preparation method includes the following steps.
(1) 393.6 mg of magnesium sulfate heptahydrate is mixed and dissolved with 4 mL of purified water to obtain a magnesium sulfate solution.
(2) 16 mg of sodium hydroxide is mixed and dissolved with 1 mL of purified water to obtain a sodium hydroxide solution.
(3) 125 mg of egg yolk phospholipid E80 is dissolved in 20 mL of dichloromethane to obtain a phospholipid solution.
(4) The phospholipid solution is transferred into a round-bottom flask, an organic solvent is removed by using a rotary evaporator to form a thin film on a flask wall, and then the thin film is hydrated with the magnesium sulfate solution to obtain a hydration solution.
(5) The sodium hydroxide solution is rapidly added into the hydration solution to obtain a mixture, then the mixture is sonicated with a probe at 220 W for 10 minutes; the magnesium hydroxide nanoparticles for treating joint pain are obtained upon completion of sonication; and it is determined by a laser particle size analyzer that an average particle size of the magnesium hydroxide nanoparticles is 539.5 nm.

### Embodiment 4

This embodiment provides a preparation method for magnesium hydroxide nanoparticles for treating joint pain, where the preparation method includes the following steps.
(1) 393.6 mg of magnesium sulfate heptahydrate is mixed and dissolved with 4 mL of purified water to obtain a magnesium sulfate solution.
(2) 32 mg of sodium hydroxide is mixed and dissolved with 1 mL of purified water to obtain a sodium hydroxide solution.
(3) 125 mg of egg yolk phospholipid E80 is dissolved in 20 mL of dichloromethane to obtain a phospholipid solution.
(4) The phospholipid solution is transferred into a round-bottom flask, an organic solvent is removed by using a rotary evaporator to form a thin film on a flask wall, and then the thin film is hydrated with the magnesium sulfate solution to obtain a hydration solution.
(5) The sodium hydroxide solution is rapidly added into the hydration solution to obtain a mixture, then the mixture is treated by a high-pressure homogenizer at 6000 psi for 5 minutes; the magnesium hydroxide nanoparticles for treating joint pain are obtained upon completion of treatment; and it is determined by a laser particle size analyzer that an average particle size of the magnesium hydroxide nanoparticles is 398.5 nm.

### Embodiment 5

This embodiment provides a preparation method for magnesium hydroxide nanoparticles for treating joint pain, where the preparation method includes the following steps.
(1) 192 mg of anhydrous magnesium sulfate is mixed and dissolved with 4 mL of purified water to obtain a magnesium sulfate solution.
(2) 8 mg of sodium hydroxide is mixed and dissolved with 1 mL of purified water to obtain a sodium hydroxide solution.
(3) 125 mg of egg yolk phospholipid E80 is dissolved in 20 mL of dichloromethane to obtain a phospholipid solution.
(4) The phospholipid solution is transferred into a round-bottom flask, an organic solvent is removed by using a rotary evaporator to form a thin film on a flask wall, and then the thin film is hydrated with the magnesium sulfate solution to obtain a hydration solution.
(5) The sodium hydroxide solution is rapidly added into the hydration solution to obtain a mixture, then the mixture is sonicated with a probe at 220 W for 10 minutes; the magnesium hydroxide nanoparticles for treating joint pain are obtained upon completion of sonication; and it is determined by a laser particle size analyzer that an average particle size of the magnesium hydroxide nanoparticles is 313.6 nm.

### Embodiment 6

This embodiment provides a preparation method for magnesium hydroxide nanoparticles for treating joint pain, where the preparation method includes the following steps.
(1) 1.2 g of magnesium sulfate heptahydrate is mixed and dissolved with 12 mL of purified water to obtain a magnesium sulfate solution.
(2) 48 mg of sodium hydroxide is mixed and dissolved with 3 mL of purified water to obtain a sodium hydroxide solution.
(3) 350 mg of soybean phospholipid S100 is dissolved in 60 mL of dichloromethane to obtain a phospholipid solution.
(4) The phospholipid solution is transferred into a round-bottom flask, an organic solvent is removed by using a rotary evaporator to form a thin film on a flask wall, and then the thin film is hydrated with the magnesium sulfate solution to obtain a hydration solution.
(5) The sodium hydroxide solution is rapidly added into the hydration solution to obtain a mixture, then the mixture is treated by a high-pressure homogenizer at 6000 psi for 5 minutes; the magnesium hydroxide nanoparticles for treating joint pain are obtained upon completion of treatment; and it is determined by a laser particle size analyzer that an average particle size of the magnesium hydroxide nanoparticles is 363.9 nm.

### Embodiment 7

This embodiment provides a preparation method for magnesium hydroxide nanoparticles for treating joint pain, where the preparation method includes the following steps.
(1) 393.6 mg of magnesium sulfate heptahydrate is mixed and dissolved with 4 mL of purified water to obtain a magnesium sulfate solution.
(2) 8 mg of sodium hydroxide is mixed and dissolved with 1 mL of purified water to obtain a sodium hydroxide solution.
(3) 60 mg of egg yolk phospholipid E80 is dissolved in 20 mL of dichloromethane to obtain a phospholipid solution.
(4) The phospholipid solution is transferred into a round-bottom flask, an organic solvent is removed by using a rotary evaporator to form a thin film on a flask wall, and then the thin film is hydrated with the magnesium sulfate solution to obtain a hydration solution.
(5) The sodium hydroxide solution is rapidly added into the hydration solution to obtain a mixture, then the mixture is sonicated with a probe at 220 W for 10 minutes; the magnesium hydroxide nanoparticles for treating joint pain are obtained upon completion of sonication; and it is determined by a laser particle size analyzer that an average particle size of the magnesium hydroxide nanoparticles is 411.7 nm.

### Embodiment 8

This embodiment provides a preparation method for magnesium hydroxide nanoparticles for treating joint pain, where the preparation method includes the following steps.
(1) 440 mg of magnesium sulfate monohydrate is mixed and dissolved with 8 mL of purified water to obtain a magnesium sulfate solution.
(2) 16 mg of sodium hydroxide is mixed and dissolved with 2 mL of purified water to obtain a sodium hydroxide solution.
(3) 200 mg of egg yolk lecithin PC98-T is dissolved in 50 mL of absolute ethyl alcohol to obtain a phospholipid solution.
(4) The phospholipid solution is transferred into a round-bottom flask, an organic solvent is removed by using a rotary evaporator to form a thin film on a flask wall, and then the thin film is hydrated with the magnesium sulfate solution to obtain a hydration solution.
(5) The sodium hydroxide solution is rapidly added into the hydration solution to obtain a mixture, then the mixture is treated by a ball mill (Emax ball mill of Retsch company in Germany) at 800 rpm for 2 h; the magnesium hydroxide nanoparticles for treating joint pain are obtained upon completion of treatment; and it is determined by a laser particle size analyzer that an average particle size of the magnesium hydroxide nanoparticles is 206.5 nm.

### Embodiment 9

This embodiment provides a preparation method for magnesium hydroxide nanoparticles for treating joint pain, where the preparation method includes the following steps.
(1) 200 mg of anhydrous magnesium chloride (Aladdin, purity 99%) is mixed and dissolved with 8 mL of purified water to obtain a magnesium chloride solution.
(2) 16 mg of sodium hydroxide is mixed and dissolved with 2 mL of purified water to obtain a sodium hydroxide solution.
(3) 200 mg of egg yolk lecithin PC98-T is dissolved in 50 mL of absolute ethyl alcohol to obtain a phospholipid solution.
(4) The phospholipid solution is transferred into a round-bottom flask, an organic solvent is removed by using a rotary evaporator to form a thin film on a flask wall, and then the thin film is hydrated with the magnesium chloride solution to obtain a hydration solution.
(5) The sodium hydroxide solution is rapidly added into the hydration solution to obtain a mixture, then the mixture is treated by a ball mill (Emax ball mill of Retsch company in Germany) at 800 rpm for 2 h; the magnesium hydroxide nanoparticles for treating joint pain are obtained upon completion of treatment; and it is determined by a laser particle size analyzer that an average particle size of the magnesium hydroxide nanoparticles is 228.1 nm.

### Embodiment 10

This embodiment provides a preparation method for magnesium hydroxide nanoparticles for treating joint pain, where the preparation method includes the following steps.
(1) 420 mg of magnesium chloride hexahydrate (Aladdin, purity 99%) is mixed and dissolved with 8 mL of purified water to obtain a magnesium chloride solution.
(2) 16 mg of sodium hydroxide is mixed and dissolved with 2 mL of purified water to obtain a sodium hydroxide solution.
(3) 200 mg of egg yolk lecithin PC98-T is dissolved in 50 mL of absolute ethyl alcohol to obtain a phospholipid solution.
(4) The phospholipid solution is transferred into a round-bottom flask, an organic solvent is removed by using a rotary evaporator to form a thin film on a flask wall, and then the thin film is hydrated with the magnesium chloride solution to obtain a hydration solution.
(5) The sodium hydroxide solution is rapidly added into the hydration solution to obtain a mixture, then the mixture is treated by a ball mill (Emax ball mill of Retsch company in Germany) at 800 rpm for 2 h; the magnesium hydroxide nanoparticles for treating joint pain are obtained upon completion of treatment; and it is determined by a laser particle size analyzer that an average particle size of the magnesium hydroxide nanoparticles is 235.0 nm.

### Embodiment 11

This embodiment provides a preparation method for magnesium hydroxide nanoparticles for treating joint pain, where the preparation method includes the following steps.
**(1)** 440 mg of magnesium sulfate monohydrate is mixed and dissolved with 8 mL of purified water to obtain a magnesium sulfate solution.
**(2)** 16 mg of sodium hydroxide is mixed and dissolved with 2 mL of purified water to obtain a sodium hydroxide solution.
(3) 800 mg of egg yolk lecithin PC98-T is dissolved in 50 mL of absolute ethyl alcohol to obtain a phospholipid solution.
(4) The phospholipid solution is transferred into a round-bottom flask, an organic solvent is removed by using a rotary evaporator to form a thin film on a flask wall, and then the thin film is hydrated with the magnesium sulfate solution to obtain a hydration solution.
(5) The sodium hydroxide solution is rapidly added into the hydration solution to obtain a mixture, then the mixture is treated by a ball mill (Emax ball mill of Retsch company in Germany) at 1000 rpm for 6 h; the magnesium hydroxide nanoparticles for treating joint pain are obtained upon completion of treatment; and it is determined by a laser particle size analyzer that an average particle size of the magnesium hydroxide nanoparticles is 65.3 nm.

### Comparative Example 1

125 mg of egg yolk phospholipid E80 is dissolved in 20 mL of dichloromethane to obtain a mixture, and then the mixture is transferred to a round-bottom flask. An organic reagent is removed by a rotary evaporator to form a thin film on a flask wall, and 5 mL of purified water is used for hydrating the thin film, making the thin film fall off to form a hydration solution, and then the hydration solution is sonicated with a probe at 220 W for 10 minutes to obtain blank liposomes.

### Comparative Example 2

(1) 393.6 mg of magnesium sulfate heptahydrate is dissolved in 4 mL of purified water to obtain a magnesium sulfate solution.
(2) 8 mg of sodium hydroxide is dissolved in 1 mL of purified water to obtain a sodium hydroxide solution.
(3) The sodium hydroxide solution is rapidly added into the magnesium sulfate solution to obtain a mixture, then the mixture is sonicated with a probe at 220 W for 10 minutes. A result shows that the system presents obvious white precipitation, and the particle size has exceeded the measurement range of the laser particle size analyzer.

### Test Example 1

An internal structure of the magnesium hydroxide nanoparticle is observed.

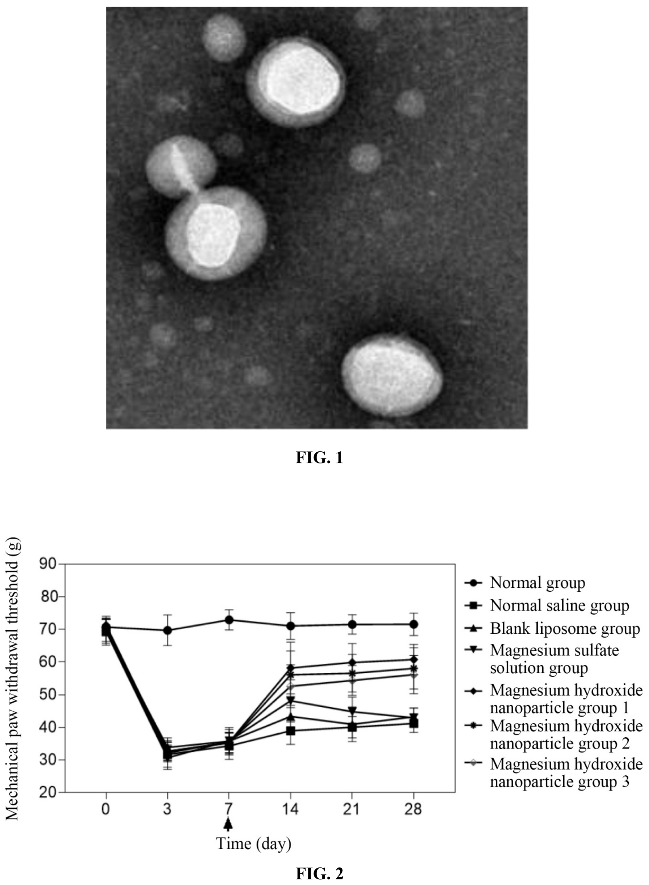

**A** structure of the magnesium hydroxide nanoparticle prepared in Embodiment 1 is observed by a transmission electron microscope (TEM). As shown in FIG. 1, the magnesium hydroxide nanoparticles prepared in the present disclosure are spherical, and magnesium hydroxide can be coated with phospholipids.

### Test Example 2

The effect of the magnesium hydroxide nanoparticle is tested.

### (1) OA rat model

Sodium iodoacetate is injected into a knee joint cavity of an SD rat to construct an OA rat model. When the knee joint of the rat swells and a pain threshold decreases significantly, the OA rat model is obtained.

### (2) Pharmacodynamic study

Through intra-articular injection, OA model rats are treated with normal saline (designated as a normal saline group), blank liposomes prepared in Comparative Example 1 (designated as a blank liposome group), a magnesium sulfate solution (a magnesium sulfate heptahydrate dissolved in the normal saline, designated as a magnesium sulfate solution group), a magnesium hydroxide nanoparticle solution prepared in Embodiment 1 (designated as a magnesium hydroxide nanoparticle group 1), a magnesium hydroxide nanoparticle solution prepared in Embodiment 3 (designated as a magnesium hydroxide nanoparticle group 2), and a magnesium hydroxide nanoparticle solution prepared in Embodiment 11 (designated as a magnesium hydroxide nanoparticle group 3).

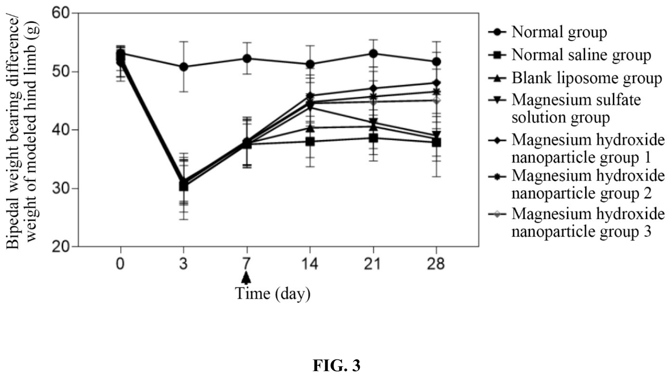

In addition, an equal volume (100 µL) of normal saline is injected into a knee joint cavity of the normal rat as a control (designated as the normal group). An administration concentration for each group is 0.4 mmol/mL (calculated as magnesium), and the administration is carried out on the 7^{-th} day after modeling, with a total of one dose. From the first day of administration, mechanical paw withdrawal thresholds and bipedal weight bearing differences of each group of rats are measured every week. The results are shown in FIG. 2 and FIG. 3, respectively.

As can be learned from results in FIG. 2 and FIG. 3, the magnesium hydroxide nanoparticles prepared in the present disclosure can effectively and permanently relieve the joint pain of the OA rat. In addition, these results fully show that the magnesium hydroxide nanoparticles provided by the present disclosure can be used for preparing a medicine for treating joint pain.

The foregoing is only the specific implementation of the present disclosure, but the scope of protection of the present disclosure is not limited thereto. Any variation or replacement that can be easily conceived by a person skilled in the art within the technical scope disclosed in the present disclosure shall fall within the scope of protection of the present disclosure. Therefore, the scope of protection of the present disclosure shall be subject to the appended claims.

## Claims

1. Magnesium hydroxide nanoparticles for treating joint pain, wherein the magnesium hydroxide nanoparticles comprise magnesium hydroxide and phospholipid.

2. The magnesium hydroxide nanoparticles for treating joint pain according to claim 1, wherein the magnesium hydroxide nanoparticle comprises a "core-shell" structure, wherein a core layer is magnesium hydroxide, and a shell layer is phospholipid.

3. The magnesium hydroxide nanoparticles for treating joint pain according to claim 1, wherein the phospholipid is one or more of natural soybean phospholipid, natural egg yolk phospholipid, and synthetic phospholipid.

4. The magnesium hydroxide nanoparticles for treating joint pain according to claim 3, wherein the phospholipid is one or a combination of multiple phospholipids selected from S45, S75, S100, SPC, E80, EPCS, EPG, SPC-3, PC98-T, PL-100M, HSPC, PGE, PGSH, DS-PL95E, DSPE, DPPA, DSPA, and DMPC.

5. The magnesium hydroxide nanoparticles for treating joint pain according to claim 4, wherein the magnesium hydroxide nanoparticles have an average particle size of 50-550 nm.

6. A preparation method for the magnesium hydroxide nanoparticles for treating joint pain according to any one of claims 1 to 5, wherein the preparation method comprises the following steps.
(1) dissolving soluble magnesium salt in purified water to obtain a magnesium salt solution;
(2) dissolving soluble metal hydroxide in purified water to obtain a metal hydroxide solution;
(3) dissolving phospholipid in an organic solvent to obtain a phospholipid solution;
(4) carrying out rotary evaporation on the phospholipid solution to remove the organic reagent, and after a thin film is formed, adding the magnesium salt solution for hydration to obtain a hydration solution; and
(5) adding the metal hydride solution into the hydration solution for nanonization treatment, and obtaining magnesium hydroxide nanoparticles for treating joint pain upon the completion of nanonization treatment.

7. The preparation method for the magnesium hydroxide nanoparticles for treating joint pain according to claim 6, wherein in step (1), the soluble magnesium salt is one or a combination of multiple selected from anhydrous magnesium sulfate, magnesium sulfate monohydrate, magnesium sulfate heptahydrate, magnesium chloride, and magnesium chloride hexahydrate.

8. The preparation method for the magnesium hydroxide nanoparticles for treating joint pain according to claim 6, wherein in step (2), the metal hydroxide is one or a combination of sodium hydroxide and potassium hydroxide.

9. The preparation method for the magnesium hydroxide nanoparticles for treating joint pain according to claim 6, wherein in step (5), a nanonization treatment mode is one of probe ultrasonic treatment, high-pressure homogenization treatment, or ball mill treatment.

10. Application of the magnesium hydroxide nanoparticles for treating joint pain according to any one of claims 1 to 5, wherein the magnesium hydroxide nanoparticles are used for preparing a medicine for treating joint pain of osteoarthritis.
